# EUROPÄISCHE PATENTSCHRIFT

(11) **EP 2 638 000 B1**
(45) Veröffentlichungstag und Bekanntmachung des Hinweises auf die Patenterteilung: **03.01.2018**
(21) Anmeldenummer: 11788090.6
(22) Anmeldetag: 07.11.2011
(51) Int. Cl.: C07C 67/03, C07C 67/08, C07C 69/58

(54) **VERFAHREN ZUR HERSTELLUNG VON CARBONSÄUREESTERN WÄHREND EINES TRANSPORTS**
PROCESS FOR PREPARING CARBOXYLIC ESTERS WHILE IN TRANSIT
PROCÉDÉ DE PRÉPARATION D'ESTERS D'ACIDES CARBOXYLIQUES PENDANT UN TRANSPORT

(30) Priorität: 11.11.2010 DE 102010050917
(43) Veröffentlichungstag der Anmeldung: 18.09.2013
(73) Patentinhaber: Emery Oleochemicals GmbH, 40599 Düsseldorf (DE)
(72) Erfinder: MÜLLER, Heinz, 40789 Monheim (DE); MÄKER, Diana, 40822 Mettmann (DE)
(74) Vertreter: Kinkeldey, Daniela
(86) Internationale Anmeldenummer: PCT/EP2011/069559
(87) Internationale Veröffentlichungsnummer: WO 2012/062709

(56) Entgegenhaltungen:
- WO-A1-2010/085864
- WO-A1-2010/093670
- WO-A2-2008/047142
- WO-A2-2008/129415

## Beschreibung

Die vorliegende Erfindung betrifft Verfahren zur Herstellung von Carbonsäureestern, welche durch Veresterung oder Umesterung von carbonsäurehaltigen Komponenten und Alkoholen erhalten werden. Das erfindungsgemässe Verfahren wird unter Verwendung eines Schiffes durchgeführt. Carbonsäureester werden in der Technik weit verbreitet genutzt. Beispielhafte Anwendungen liegen im Bereich der Schmiermittel und der Trägermaterialien für kosmetische Produkte. So offenbart beispielsweise die DE 10 2005 052 173 A1 die Verwendung von Fettsäure-2-Ethylhexylestergemischen als Ölkomponente in kosmetischen und pharmazeutischen Produkten.

Zahlreiche Verfahren sind bekannt, um Carbonsäureester aus carbonsäurehaltigen Komponenten und Alkoholen herzustellen. Diesen Verfahren ist gemein, dass carbonsäurehaltige Komponente und Alkohol separat zum Ort der technischen Veresterung oder Umesterung transportiert und erst am Ort der technischen Veresterung miteinander in Kontakt gebracht werden. Hierbei ist besonders der Schritt der Umladung der carbonsäurehaltigen Komponente aufgrund ihrer häufig hohen Viskosität problematisch, der in üblichen Verfahren mindestens zweimal durchlaufen wird.

Bei einigen carbonsäurehaltigen Komponenten mit hohem Schmelzpunkt ist ein der Umladung oder der Umsetzung vorhergehendes Aufschmelzen notwendig, wodurch der Aufwand dieser Schritte zusätzlich steigt. Beispiele für solche carbonsäurehaltige Komponenten sind reine Carbonsäuren, wie Oxalsäure, die beim Aufschmelzen zum Zersetzen neigt, Fumarsäure, Salicylsäure, Palmitinsäure, Margarinsäure, Stearinsäure und Arachinsäure, deren Schmelzpunkt oberhalb von 50°C liegt. Aber auch Ester langkettiger Carbonsäuren mit mehrwertigen Alkoholen liegen bei Raumtemperatur in fester Form vor. Beispielhaft ist an dieser Stelle das Glyzerintrilaurat mit einem Schmelzpunkt von 46,5° C angeführt.

Ein weiterer Nachteil dieses zweistufigen Verfahrens (erste Stufe: getrennter Transport der Edukte zum Ort der Veresterung; zweite Stufe: in Kontakt bringend er Edukte am Ort der Veresterung) begründet sich durch die niedrige Geschwindigkeit der Umesterungs- oder Veresterungsreaktion. Um der Kinetik der Reaktion entgegenzuwirken und angemessene Verweilzeiten in Prozessanlagen zu realisieren, wird die Veresterung oder Umesterung in derzeit gebräuchlichen Verfahren bei hohen Temperaturen durchgeführt und mit Hilfe von Katalysatoren zusätzlich beschleunigt. Beispielhaft ist an dieser Stelle ein Verfahren aus der DE 102 51 984 A1 angeführt, das eine Umsetzung bei 190-240° C in Anwesenheit eines Tetraalkyltitanat-Katalysators in 18 Stunden vorsieht. Niedrigere Temperaturen führen zu deutlich längeren Verweilzeiten, die in herkömmlichen Prozessanlagen nicht toleriert werden können.

WO 2008/047142 A2 offenbart eine Synthese für Biodiesel. WO 2010/093670 A1 und WO 2010/085864 A1 offenbaren eine mobile Biodieselherstellungsanlage. WO 2008/129415 A2 offenbart eine Biobrennstoffherstellung und Transportanlage.

Der vorliegenden Erfindung lag die Aufgabe zugrunde, die sich aus dem Stand der Technik ergebenden Nachteile im Zusammenhang mit der Herstellung von Carbonsäureestern aus einer carbonsäurehaltigen Komponente und einem Alkohol durch Veresterung oder Umesterung zu überwinden.

Insbesondere lag der vorliegenden Erfindung die Aufgabe zugrunde, den gesamten Verfahrensprozess, beginnend mit dem Transport der Edukte bis hin zu deren Umsetzung effizienter zu gestalten.

Einen Beitrag zur Lösung der eingangs genannten Aufgaben leistet daher ein Verfahren zur Herstellung von Carbonsäureestern beinhaltend die Verfahrensschritte:
a. Bereitstellen einer carbonsäurehaltigen Komponente und eines Alkohols;
b. In Kontakt bringen der carbonsäurehaltigen Komponente und des Alkohols in einem Behältnis zu einer Umsetzung zu Carbonsäureestern durch Veresterung oder Umesterung,
dadurch gekennzeichnet, dass das Behältnis während der Veresterung oder Umesterung über eine Distanz von mindestens 1 Kilometer bewegt wird. Überraschenderweise hat sich gezeigt, dass sich die vorstehend genannten Aufgaben durch die Zusammenlegung des Transports- und des Umsetzungsschritts zu einem einstufigen Prozess lösen lassen. Insbesondere lässt sich die Handhabung besonders solcher carbonsäurehaltiger Komponenten, die bei Raumtemperatur (20°C) in festem Aggregatzustand oder als hochviskose Flüssigkeit vorliegen, durch Veresterung oder Umesterung während des Transportes deutlich verbessern. Positive Effekte werden besonders bei carbonsäurehaltigen Komponenten mit einem Schmelzpunkt oberhalb von 10°C oder auch oberhalb 20°C und ebenso oberhalb 30°C beobachtet. Die Fließ- und Pumpfähigkeit wird gegenüber der reinen carbonsäurehaltigen Komponente bereits bei einer partiellen Umsetzung zu mindestens 10 %, bezogen auf die Stoffmenge der carbonsäurehaltigen Komponente, erhöht Die Wirkungsweise hängt dabei von der genaueren Ausgestaltung der carbonsäurehaltigen Komponente ab.

Carbonsäureester sind an sich bekannte Verbindungen, die abgesehen von dem erfindungsgemäßen Verfahren nach bekannten Methoden der synthetischen Chemie aus carbonsäurehaltigen Komponenten und Alkoholen hergestellt werden können. Handelt es sich bei der carbonsäurehaltigen Komponente um eine reine Carbonsäure, so wird diese Umsetzung als "*Veresterung*" bezeichnet. Handelt es sich bei der carbonsäurehaltigen Komponente jedoch um eine Zusammensetzung, welche gegebenenfalls neben Carbonsäuren auch Carbonsäureester beinhaltet, so kann neben der Veresterung auch eine "*Umesterung*" erfolgen.

Im Verfahrensschritt a. des erfindungsgemäßen Verfahrens werden eine carbonsäurehaltige Komponente und ein Alkohol bereitgestellt.

Wird als carbonsäurehaltige Komponente ein Ester eines mehrwertigen Alkohols mit zwei oder mehr Carbonsäuren eingesetzt, so wird bei einer Umesterung dieser Ester gespalten und Carbonsäureester bestehend aus der Carbonsäure der carbonsäurehaltigen Komponente und dem eingesetzten Alkohol erhalten. Handelt es sich beim eingesetzten Alkohol um einen einwertigen Alkohol, so wird durch diesen Prozess die Größe des Moleküls und damit auch Schmelzpunkt und Viskosität reduziert.

Wird als carbonsäurehaltige Komponente ein Ester eines einwertigen Alkohols mit einer Carbonsäure eingesetzt, so kann bei einer Umesterung der Alkohol so ausgetauscht werden, dass Kettenlänge und/oder Polarität reduziert werden. Dies setzt Schmelzpunkt und Viskosität herab.

In einer erfindungsgemäßen Ausfuhrungsform wird als carbonsäurehaltige Komponente eine reine Carbonsäure oder deren Gemische mit anderen Carbonsäuren eingesetzt. Bei den Carbonsäuregemischen handelt es sich meist um technisch leicht und preiswert verfügbare Qualitäten. Beispiele hierfür sind C14 bis C18 Carbonsäuren, vorzugsweise mit einem Anteil dieser Carbonsäuren von mehr als 60 Gew.-% und bevorzugt mehr 70 Gew.-% an einem Carbonsäuegemisch. Hier sind beispielhaft Spaltfettsäuren von Kokos-, Palmkern- und Plamöl zu nennen. Da die Carbonsäureester reduzierte Schmelzpunkte und Viskositäten in Relation zur konstituierenden Carbonsäure aufweisen, erleichtert auch eine Veresterungsreaktion die Handhabung gegenüber der Carbonsäure. Bevorzugt sind hier lineare und verzweigte, gesättigte und ungesättigte Carbonsäuren mit 7 bis 30 Kohlenstoffatomen.

Als Carbonsäure kommen insbesondere Fettsäuren in Frage. Hier seien als bevorzugt genannt die Capronsäure, Caprylsäure, Pelargonsäure, 2-Ethylhexansäure, Caprinsäure, Laurinsäure, Isotridecansäure, Myristinsäure, Palmitinsäure, Palmoleinsäure, Margarinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure, Lignocerinsäure, Cerotinsäure, Montansäure, Melissinsäure und Erucasäure oder mindestens zwei davon sowie deren technische Mischungen. Besonders bevorzugt sind solche Fettsäuren mit 12 bis 18 Kohlenstoffatomen.

Als Alkohol wird im Verfahrensschritt a. vorzugsweise ein einwertiger Alkohol eingesetzt, wobei dieser Alkohol vorzugsweise 1 bis 16 Kohlenstoffatome, besonders bevorzugt 2 bis 12 Kohlenstoffatome und am meisten bevorzugt 4 bis 8 Kohlenstoffatome aufweist. Als Beispiele für erfindungsgemäß geeignete Alkohole seien insbesondere Methanol, 1-Propanol, 2-Propanol, 1-Butanol, 2-Butanol, tert.-Butanol, Propylheptylalkohol, Isotridecyalkohol, Lineare-C6-, -C8-, -C10-, Propylheptylalkohol, Isotridecyalkohol, Lineare-C6-, -C8-, -C10-Alkohole und Ethylhexanol sowie Mischungen aus mindestens zwei davon genannt.

Im Verfahrensschritt b. des erfindungsgemäßen Verfahrens werden die carbonsäurehaltige Komponente und der Alkohol in einem Behältnis zu einer Umsetzung zu Carbonsäureestern durch Veresterung oder Umesterung in Kontakt gebracht, wobei das Behältnis während der Veresterung oder Umesterung über eine Distanz von mindestens 1 Kilometer bewegt wird.

Als Behältnis, in dem carbonsäurehaltige Komponente und Alkohol in Kontakt gebracht werden, kommen sämtliche Behältnisse in Frage, die dazu geeignet sind, carbonsäurehaltige Komponenten, Alkohole und Carbonsäureester zu beinhalten. Typischerweise werden für Flüssigkeiten und schmelzbare Feststoffe dieser Art Container oder Fässer aus nicht rostendem Stahl verwendet. Es ist jedoch ebenso möglich, Kunststoffbehälter, oft aus Polyethylen, Polypropylen oder Polyester oder einer Mischung aus mindestens zwei davon.

Die Bewegung des Behältnisses dient dem Zweck, die carbonsäurehaltige Komponente vom Ort der Herstellung oder des Verkaufs (A) zu dem Ort zu transportieren, an dem sie als Carbonsäureester oder als Gemisch des Carbonsäureesters mit den Edukten einer Bestimmung zugeführt wird (B). Zweckmäßig sind daher Bewegungen, die die Orte A und B annähernd linear unter Berücksichtigung geographischer, infrastruktureller und sonstiger Gegebenheiten verbindet. Erfindungsgemäss wird das Behältnis durch einen Schifftransport bewegt. Durch die Nutzung der Transportzeit zur Umsetzung von carbonsäurehaltiger Komponente und Alkohol sind lange Reaktionszeiten und somit niedrige Reaktionstemperaturen im Bereich von 35 bis 65° C tolerierbar. Dies ermöglicht den Einsatz alternativer Verfahren zur Erwärmung des Behältnisses. Bevorzugt sind daher auch solche Ausgestaltungen, die es ermöglichen, die Abwärme eines Schiffsmotors zu nutzen, um das Reaktionsbehältnis zu erwärmen. Dies kann zum Beispiel durch eine Positionierung des Behältnisses in räumlicher Nähe zu einem Schiffsmotor oder auch durch Weiterleitung der von einem Motor erzeugten Wärme mit Hilfe einer Wärmeleitflüssigkeit zum Behältnis erfolgen.

Bevorzugt sind des Weiteren solche Ausgestaltungen, in denen das Reaktionsbehältnis bereits durch die klimatischen Bedingungen während des Bewegens erwärmt wird. Dies wird erreicht, indem das Reaktionsbehältnis in Regionen mit erhöhten Lufttemperaturen und/oder starker Sonneneinstrahlung bewegt wird. Diese Bedingungen finden sich beispielhaft in den Regionen tropischen Klimas zwischen dem nördlichen und dem südlichen Wendekreis.

Der Zeitraum, in dem das Behältnis bewegt wird, oder die Distanz, über die es bewegt wird, sind an sich nicht begrenzt. Carbonsäureester sind stabile chemische Verbindungen, die keiner direkt an ihre Herstellung anschließende Aufbereitung oder Weiterverarbeitung bedürfen. Bevorzugt sind solche Ausführungen, in denen das Behältnis für mindestens 3, vorzugsweise mindestens 7, Tage oder mindestens über eine Distanz von 100 km, bevorzugt 500 km und besonders bevorzugt 1500 km und besonders bevorzugt 5000 km bewegt wird.

In einer erfindungsgemäßen Ausführungsform werden carbonsäurehaltige Komponente und Alkohol in technisch üblicher Reinheit eingesetzt. Der Wassergehalt der Reaktionsmischung soll daher 10 Gew.-%, bezogen auf die Reaktionsmischung, nicht überschreiten.

Aufgrund der Tolerierbarkeit langer Reaktionszeiten kann der Einsatz von Katalysatoren wie er sonst bei der Herstellung von Carbonsäureestern üblich ist auf weniger als 20 Gew.-% Katalysator, bezogen auf die Reaktionsmischung, beschränkt werden.

Einen Beitrag zur Lösung der eingangs genannten Aufgaben leistet auch die Verwendung eines Schiffes als Ort der Herstellung eines Carbonsäureesters durch in Kontakt bringen einer carbonsäurehaltigen Komponente und eines Alkohols in einem auf dem Schiff befindlichen Behältnis, wobei das in Kontakt bringen und somit die Veresterung oder Umesterung vorzugsweise während der Fahrt des Schiffes erfolgt.

Die Erfindung wird nun anhand nicht limitierender Beispiele näher erläutert.

### BEISPIEL

Ein erfindungsgemäßes Verfahren wurde bei der Umsetzung eines Fettsäuregemisches mit Ethylhexanol angewendet. Das Fettsäuregemisch verfügte über einen hohen Anteil an Ölsäure und war bei Raumtemperatur eine trübe viskose Flüssigkeit mit Bodensatz. 2 kg des Fettsäuregemisches wurden 0,5 kg Ethylhexanol zugesetzt und für 4 Wochen bei 40°C auf bewegt, ohne die Durchmischung aktiv durch ein Rührwerk zu unterstützen. Die Umsetzung der Carbonsäure wurde über die Säurezahl quantifiziert.

| **Zeit [Wochen]** | **Säurezahl [mg KOH/g]** | **Viskosität** |
|---|---|---|
| 0 | 144,3 | hoch mit Bodensatz |
| 2 | 78,0 | hoch |
| 4 | 68,8 | mittel |

Es zeigte sich, dass die Carbonsäure zu 68,8 %, bezogen auf die Stoffmengen der Carbonsäure, umgesetzt wird. Durch die Veresterung wird die Viskosität reduziert und der Bodensatz aufgelöst.

## Patentansprüche

1. Ein Verfahren zur Herstellung von Carbonsäureestern beinhaltend die Verfahrensschritte:
a. Bereitstellen einer carbonsäurehaltigen Komponente und eines Alkohols;
b. In Kontakt bringen der carbonsäurehaltigen Komponente und des Alkohols in einem Behältnis zu einer Umsetzung zu Carbonsäureestern durch Veresterung oder Umesterung,
wobei das Behältnis während der Veresterung oder Umesterung über eine Distanz von mindestens 1 Kilometer bewegt wird und wobei das Bewegen über einen Zeitraum von mindestens 7 Tagen erfolgt, wobei das Bewegen des Behältnisses durch einen Schifftransport erfolgt,
wobei das Behältnis ein Container aus nicht rostendem Stahl, ein Fass aus nicht rostendem Stahl oder ein Kunststoffbehälter ist, und
die carbonsäurehaltige Komponente eine Carbonsäure oder deren Gemische mit anderen Carbonsäuren oder eine Zusammensetzung ist, die Carbonsäuren und Carbonsäureester beinhaltet.

2. Verfahren nach Anspruch 1, wobei der Schmelzpunkt der carbonsäurehaltigen Komponente oberhalb von 10° C liegt.

3. Verfahren nach einem der vorhergehenden Ansprüche, wobei es sich bei dem Alkohol um einen einwertigen Alkohol handelt.

4. Verfahren nach einem der vorhergehenden Ansprüche, wobei die Carbonsäure eine Carbonsäure mit 7 bis 30 Kohlenstoffatomen ist.

5. Verfahren nach einem der nach einem der vorhergehenden Ansprüche, wobei als Carbonsäure eine Fettsäure eingesetzt wird.

6. Verfahren nach Anspruch 5, wobei die Fettsäure 12 bis 18 Kohlenstoffatome enthält.

7. Verfahren nach einem der vorhergehenden Ansprüche, wobei die Reaktionsmischung durch die Abwärme eines Schiffsmotors erwärmt wird.

8. Verfahren nach einem der Ansprüche 1 bis 7, wobei das Reaktionsbehältnis durch Klimabedingungen beim Bewegen erwärmt wird.

9. Verfahren nach einem der vorhergehenden Ansprüche, wobei das Behältnis während der Veresterung mindestens über eine Distanz von 100 km bewegt wird.

10. Verfahren nach einem der vorhergehenden Ansprüche, wobei die Umsetzung zu mindestens 10 %, bezogen auf die Stoffmenge der carbonsäurehaltigen Komponente, erfolgt.

11. Verfahren nach einem der vorhergehenden Ansprüche, wobei das Bewegen bei einer relativen Luftfeuchtigkeit in einem Bereich von 10 bis 100 % erfolgt.

12. Verfahren nach einem der vorhergehenden Ansprüche, wobei das Bewegen bei einer Temperatur in einem Bereich von 35 bis 65 °C erfolgt.

13. Verfahren nach einem der vorhergehenden Ansprüche, wobei der Anteil an Wasser an der Reaktionsmischung 10 Gew.-%, bezogen auf die Reaktionsmischung, nicht überschreitet.

14. Verfahren nach einem der vorhergehenden Ansprüche, wobei die Umsetzung mit weniger als 20 Gew.-% Katalysator, bezogen auf die Reaktionsmischung, erfolgt.

## Claims

1. A process for production of carboxylic esters comprising the steps of:
a. providing a carboxylic acid component and an alcohol;
b. bringing the carboxylic acid component and the alcohol into contact in a receptacle for conversion into carboxylic esters by esterification or transesterification,
wherein the receptacle is moved over a distance of at least 1 kilometre during the esterification or transesterification and wherein the movement takes place over a period of at least 7 days, wherein the movement of the receptacle is effected by transportation on a ship,
wherein the receptacle is a container composed of stainless steel, a barrel composed of stainless steel or a plastics container, and
the carboxylic acid component is a carboxylic acid or mixtures thereof with other carboxylic acids or is a composition comprising carboxylic acids and carboxylic esters.

2. Process according to claim 1, wherein the melting point of the carboxylic acid component is above 10°C.

3. Process according to any one of the preceding claims, wherein the alcohol is a monohydric alcohol.

4. Process according to any one of the preceding claims, wherein the carboxylic acid is a carboxylic acid having 7 to 30 carbon atoms.

5. Process according to any one of the preceding claims, wherein the carboxylic acid used is a fatty acid.

6. Process according to claim 5, wherein the fatty acid contains 12 to 18 carbon atoms.

7. Process according to any one of the preceding claims, wherein the reaction mixture is heated by the exhaust heat of a ship engine.

8. Process according to any one of claims 1 to 7, wherein the reaction receptacle is heated by climatic conditions during movement.

9. Process according to any one of the preceding claims, wherein the receptacle is moved over at least a distance of 100 km during the esterification.

10. Process according to any one of the preceding claims, wherein the conversion is at least 10%, based on the amount-of-substance of the carboxylic acid component.

11. Process according to any one of the preceding claims, wherein the movement is effected at a relative humidity in the range from 10 to 100%.

12. Process according to any one of the preceding claims, wherein the movement is effected at a temperature in the range from 35 to 65°C.

13. Process according to any one of the preceding claims, wherein the proportion of water in the reaction mixture does not exceed 10 wt%, based on the reaction mixture.

14. Process according to any one of the preceding claims, wherein the conversion is effected using less than 20 wt% of catalyst, based on the reaction mixture.

## Revendications

1. Procédé pour la production d'esters d'acides carboxyliques, comprenant les étapes de processus :
a. fourniture d'un composant contenant un acide carboxylique et d'un alcool ;
b. mise en contact du composant contenant un acide carboxylique et de l'alcool dans un récipient, entraînant une réaction conduisant à des esters d'acides carboxyliques par estérification ou transestérification,
pendant l'estérification ou la transestérification le récipient étant déplacé sur une distance d'au moins 1 kilomètre et le déplacement s'effectuant pendant une durée d'au moins 7 jours, le déplacement du récipient s'effectuant par un transport par bateau,
le récipient étant un conteneur en acier inoxydable, un fût en acier inoxydable ou un récipient en plastique, et
le composant contenant un acide carboxylique étant un acide carboxylique ou ses mélanges avec d'autres acides carboxyliques ou une composition qui contient des acides carboxyliques et des esters d'acides carboxyliques.

2. Procédé selon la revendication 1, le point de fusion du composant contenant un acide carboxylique étant supérieur à 10 °C.

3. Procédé selon l'une quelconque des revendications précédentes, l'alcool consistant en un alcool monohydrique.

4. Procédé selon l'une quelconque des revendications précédentes, l'acide carboxylique étant un acide carboxylique ayant de 7 à 30 atomes de carbone.

5. Procédé selon l'une quelconque des revendications précédentes, dans lequel on utilise comme acide carboxylique un acide gras.

6. Procédé selon la revendication 5, l'acide gras comportant de 12 à 18 atomes de carbone.

7. Procédé selon l'une quelconque des revendications précédentes, le mélange réactionnel étant chauffé par la chaleur émise par un moteur de bateau.

8. Procédé selon l'une quelconque des revendications 1 à 7, le récipient de réaction étant chauffé par des conditions climatiques lors du déplacement.

9. Procédé selon l'une quelconque des revendications précédentes, pendant l'estérification le récipient étant déplacé au moins sur une distance de 100 km.

10. Procédé selon l'une quelconque des revendications précédentes, la réaction s'effectuant à raison d'au moins 10 %, par rapport à la quantité de substance du composant contenant un acide carboxylique.

11. Procédé selon l'une quelconque des revendications précédentes, le déplacement s'effectuant sous une humidité relative de l'air dans une plage de 10 à 100 %.

12. Procédé selon l'une quelconque des revendications précédentes, le déplacement s'effectuant à une température dans une plage de 35 à 65 °C.

13. Procédé selon l'une quelconque des revendications précédentes, la teneur en eau du mélange réactionnel n'excédant pas 10 % en poids, par rapport au mélange réactionnel.

14. Procédé selon l'une quelconque des revendications précédentes, la réaction s'effectuant avec moins de 20 % en poids de catalyseur, par rapport au mélange réactionnel.
